# EUROPEAN PATENT APPLICATION

(11) **EP 3 915 575 A1**
(43) Date of publication of application: **01.12.2021**
(21) Application number: 20177311.6
(22) Date of filing: 29.05.2020
(51) Int. Cl.: A61K 39/00

(54) **VACCINE FORMULATIONS**

(71) Applicant: IMNATE SARL, 8009 Strassen (LU)
(72) Inventor: SAINT-REMY, Jean-Marie, 1390 Grez-Doiceau (BE)
(74) Representative: Office Kirkpatrick

(57) **Abstract**

A method to elicit CD4+ T cells with an immune suppressive function towards a given MHC Class II epitope present in a specific target tissue and/or to elicit CD4+ T cells harbouring homeostasis restoration properties for a specific target tissue, based on an MHC Class II epitope modified by the addition of a redox motif, the corresponding modified epitopes, pharmaceutical uses, kits of parts, and gene edition system for use in a patient.

## Description

### Field of the invention

The present invention relates to new vaccination compositions based on modified epitopes, useful for the specific treatment of unwanted immune responses, as well as for enhancing tissue repair.

### BACKGROUD OF THE INVENTION

Autoimmune diseases are characterized by the production of antibodies and activation of lymphocytes directed towards self-antigens, leading to the progressive loss of function of the target organ.
Although there is clear evidence for the pathogenic role of autoantibodies and autoreactive immune cells in the triggering and maintenance of autoimmune diseases, supported by the relative efficacy of therapies based on non-specific immunosuppression or administration of antibodies targeting cytokines, there is no cure for such diseases. This, combined to the steadily raising incidence of autoimmunity, constitutes a highly significant unmet medical need. Indeed, strategies by which it would become possible to suppress the autoimmune response without affecting the overall immune system are much desired.

Currently, a limited number of strategies have been defined in an attempt to selectively suppress the autoimmune response.
However, these approaches are very complex in practice, sometimes associated only to a transient effect and the demonstration of their significant usefulness is sometimes lacking.
The patent application WO2008/017517 A1 (Immunogenic peptides and their use in immune disorders) describes peptides and methods wherein class II MHC epitopes containing a redox (thioreductase) motif C-X-X-C (wherein C stands for cysteine and X for any aminoacid) are used for eliciting epitope-specific CD4+ T cells with cytolytic properties (Foxp3+ CD4+ T cells). Elimination by cytolysis of the activating APC and of bystander T cells is said to be efficient for the treatment of immune disorders, and in particular autoimmune and allergic diseases. These peptides contain a thioreductase motif, which is attached by a covalent amide linkage (peptide bond), on either side of the epitope sequence, with or without an aminoacid linker. Due to the open end structure of MHC class II (MHC2) molecules, it is indeed possible to use peptides much longer than what would be allowed if length would be limited by the sequence inserted into the cleft of the class II element. However, this patent application does not disclose or even suggest a role *in vivo* for an action on CD4+ T cells with a suppressive function (Foxp3-), which is particularly advantageous for the treatment of inflammation or for improving tissue repair.

T lymphocytes remain the key cells at the start of an autoimmune response or of tissue specific inflammation. Antigen-specific T cells are divided in three separate lineages, defined by the restriction element by which they are activated. CD4+ T cells are elicited in the context of presentation by MHC class II complexes, CD8+ T cells are activated through MHC class I presentation and natural killer T (NKT) cells are activated by presentation by the MHC-like CD1 molecule.
Antigen-presenting cells (APCs) when exposed to an antigen, or an epitope of it, process the antigen and expose it at their surface for specific T cell activation in a scenario which is classically described in 3 steps: (1) contact between a T cell via its antigen-specific receptor (CD3) with the antigen epitope as processed by the APC and presented in the context of an MHC molecule (signal 1); (2) interaction between the costimulatory signals expressed at the APC surface and their respective ligand or receptor at the T cell surface (signal 2); and, (3) production of soluble factors including cytokines and chemokines by the APC (signal 3).
In the setting of autoimmune diseases, or tissue-associated chronic inflammation, a vaccination strategy aiming at suppressing the unwanted response takes these signals into account. In short, intrinsic tolerance is obtained primarily in the absence of an adjuvant, whilst extrinsic tolerance is obtained by manipulating the cytokine milieu under which activation occurs.
However, these methods are not versatile or potent enough to treat complex diseases.

On the other hand, some of the cells of the immune system belonging to the T cell lineage exert strong suppressive properties on immune responses, but carry also a number of additional properties including anti-inflammatory, tissue repair and tissue regeneration, pro-angiogenesis and metabolic regulation.

Two types of such cells have been described:
Regulatory T cells belong to the adaptive immune system, carrying an antigen-specific receptor, selected centrally in the thymus and/or in the periphery. Such cells are known to be essential for preventing auto-immune responses. Many subsets of regulatory T cells have been described with varying phenotypes and functional properties, and the capacity to change function and phenotype according to specific tissue environment. However, despite intensive research, it remains difficult to elicit antigen-specific regulatory T cells with stable properties for therapeutic intervention.

Innate lymphoid cells (ILCs) are considered as sentinels of tissue immunity, carrying properties largely depending on the subset to which they belong and on the tissue concerned. Such cells do not carry an antigen-specific receptor and can therefore not be stimulated in an antigen-specific manner.

The patent application EP19182807.8 filed on June 27, 2019 describes a new powerful and easy-to-implement approach based on the addition of reducing agents together with an epitope, which is useful to treat autoimmune diseases; these compounds can be formulated into a vaccine and also allows more flexibility for the injection locus.

In an alternative, or in addition to this, the inventor has now identified the possibility to directly elicit CD4+ T cells with a strong suppressive activity on immune response (Foxp3(-)), including inflammation, and even triggering tissue repair.

### Brief description of the invention

The present patent application relates to a method, advantageously, an *in vitro* or *ex vivo* method, to elicit CD4+ T cells with an immune suppressive function towards a given MHC Class II epitope present in a specific target tissue and/or CD4+ T cells harbouring homeostasis restoration properties for a specific target tissue comprising the steps of: identifying a MHC Class II epitope from this tissue associated to (affected by) an unwanted immune disease; of generating a modified MHC Class II epitope comprising at least one additional redox motif adjacent to this MHC Class II epitope or separated to this MHC Class II epitope by a linker of up to 7 amino acids, and of contacting (*in vitro*) CD4+ T cells with this modified MHC Class II epitope.

In this method, preferably, the CD4+ T cells are contacted with a pharmaceutically-acceptable antioxidant together with the modified MHC Class II epitope.

Preferably, this method further comprises the step of characterizing and/or of sorting and/or of isolating the CD4+ T cells (Foxp3(-)) for their expression of surface markers selected from the group consisting of TIGIT, DLL4 and CTLA2, and/or for their secretion of soluble factors selected from the group consisting of Interleukin (Il)-10, IL-13, IL-4, transforming growth factor (TGF) β-2, amphiregulin, arginase and meteorin.

A related aspect of the present invention is the CD4+ T cells obtainable by this method, as well as their use as a medicament, preferably in the treatment of an unwanted immune response and/or in tissue repair.

A related aspect of the present invention is a modified MHC Class II epitope comprising at least one additional redox motif adjacent to a MHC Class II epitope or separated to the said MHC Class II epitope by a linker of up to 7 amino acids, for use in the localized production of cytokines with a suppressive function in a tissue of a patient having developed an unwanted immune response towards a peptide bearing the said MHC Class II epitope or bearing a substantially similar MHC Class II epitope, preferably wherein this production of cytokines is (mainly) localized in (or close to) the tissue affected by this unwanted immune response, more preferably wherein these cytokines are selected from the group consisting of Interleukin (Il)-10, IL-13, IL-4, transforming growth factor (TGF) β-2, and possibly also selected from amphiregulin, arginase and meteorin, still more preferably wherein the amount of these cytokines (at least Il-10 and/or IL-13 and/or IL-4) is at least doubled (multiplied by a factor 3, 5, 10, 30, 50 or even 100) in the affected tissue after the administration of the MHC Class II epitope comprising the additional redox motif and/or more preferably, wherein the amount of these cytokines in other tissues of the patients (not affected by this unwanted immune response), also possibly in a blood sample obtained from another part of the patient's body than the affected tissue, or close to the affected tissue, is not significantly increased (less than increased by 10 fold, less than increased by 5 fold, or even less than doubled) after the administration of the MHC Class II epitope comprising the additional redox motif.

This is advantageous for the conversion in a specific tissue of the patient of inflammatory macrophages into non-inflammatory macrophages, preferably in the adipose tissue.

Another related aspect of the present invention is thus a modified MHC Class II epitope comprising at least one additional redox motif adjacent to a MHC Class II epitope or separated to the said MHC Class II epitope by a linker of up to 7 amino acids, for use in tissue repair in a patient suffering from an unwanted immune disease (or from an inflammatory disease, caused by an unwanted immune response) towards a peptide bearing the said MHC Class II epitope or bearing a substantially similar MHC Class II epitope.

Another related aspect of the present invention is a kit of parts comprising (i) a modified MHC Class II epitope comprising at least one additional redox motif adjacent to a MHC Class II epitope or separated to the said MHC Class II epitope by a linker of up to 7 amino acids for use in the treatment of an unwanted immune response towards a peptide bearing the said MHC Class II epitope or bearing a substantially similar MHC Class II epitope, and (ii) means to monitor the activation of (Foxp3(-)) CD4+ T cells, wherein the said MHC Class II epitope is expressed in a patient's tissue and/or organ affected by the said unwanted immune response and preferably wherein the means to monitor the activation of (Foxp3(-)) CD4+ T cells comprise (*in vitro*) means to monitor the activation of (Foxp3(-)) CD4+ T cells within the said affected tissue.

In this kit of parts, the means to monitor the activation of (Foxp3(-)) CD4+ T cells are preferably means for measuring the expression by this (Foxp3(-)) CD4+ T cells of surface markers selected from the group consisting of TIGIT, DLL4 and CTLA2, and/or means to monitor the secretion by the said (Foxp3(-)) CD4+ T cells of soluble factors selected from the group consisting of Interleukin (Il)-10, IL-13, IL-4, transforming growth factor (TGF) β-2, amphiregulin, arginase and meteorin.

Preferably, this kit of parts further comprises an antioxidant compound to be administered to a patient together with the modified MHC Class II epitope.

Possibly, in the present invention (all the above aspects) the unwanted immune response is inflammation.

Another related aspect of the present invention is a modified MHC Class II epitope comprising at least one additional redox motif adjacent to a MHC Class II epitope or separated to the said MHC Class II epitope by a linker of up to 7 amino acids for use in eliciting (Foxp3(-)) CD4+ T cells expressing surface markers selected from the group consisting of TIGIT, DLL4 and CTLA2, and/or secreting (measurable amounts of) soluble factors selected from the group consisting of Interleukin (Il)-10, IL-13, IL-4, transforming growth factor (TGF) β-2, amphiregulin, arginase and meteorin, the said CD4+ T cells being specific for the said MHC Class II epitope, the said MHC Class II epitope being expressed in a patient's tissue and/or organ affected by an unwanted immune response.

Another related aspect of the present invention is a kit of parts comprising a gene editing system with a specific nuclease or a nucleic acid molecule encoding the said specific nuclease, and a peptide fragment of the said specific nuclease being a MHC Class II epitope, the said peptide fragment further comprising a redox motif adjacent to the said fragment of the said specific nuclease or separated to the said fragment of the said specific nuclease by a linker of up to 7 amino acids, or a nucleic acid molecule encoding the said modified peptide fragment.

This kit of parts is advantageously for use in the treatment of a genetic disease and/or the prevention and/or treatment of a viral disease upon *in situ* (*in vivo*) generation of a mutation in a cell (of the patient) targeted by the virus (generation of mutations conferring resistance, modification of gene expression), and/or for the treatment of cancer (correction of deleterious mutations in cancer cells, such as correction of mutation in p53), while not triggering an immune response of the patient towards the administered system for gene edition.

In this kit, preferably, the nuclease (for gene edition) is selected from the group consisting of TALEN, Cas, Cpf, and CSM.

In all the above aspects, in the above methods, MHC Class II epitope, CD4+ T cell, pharmaceutical composition or kit of parts according, the redox motif comprises or consists essentially of the peptide sequence CXXC, preferably a redox motif selected from the group consisting of CPXC, CXPC, HCXXC, CXXCH, HCPXC, CPCXH, HCXPC and CXPCH, wherein C stands for Cysteine or selenocysteine, X stands for any amino acid, P for proline and H for histidine.

### DETAILED DESCRIPTION OF THE INVENTION

The inventor has pioneered several methods to fine-tune the immune response, particularly by the specific targeting of T cell lineages CD4, CD8 and/or NKT.
The inventor has made the unexpected discovery that the addition of a redox motif (CXXC) in the region flanking a class II epitope (MHC 2 epitope) can be used to elicit CD4+ T cells with a direct suppressive activity, due to a unique cytokine profile including IL-10, Il-4 and IL-13, but also ROR-α (NR1F1).
This represents a specific approach to target an unwanted immune response, which is particularly advantageous in the case of inflammation or to improve tissue repair. Indeed, the inventor has found that these CD4+ cells are mobilized at the proximity of the affected tissue, which means a more potent effect and less side-effects.

In particular, addition of a motif with redox properties covalently coupled to the MHC Class II epitope reliably elicit, upon administration, preferably with an adjuvant, stable antigen-specific Foxp3 (-) CD4+ T cells with variable association of properties including:
Reduction of inflammation by production of IL-13 acting on monocytes to reduce the production of IL-6, IL-1a and LIF;
Reduction of inflammation by expression of ROR-α (NR1F1) suppressing the transcription of IL-1b, TNF, IL-6 and MCP-1;
Attracting and conditioning myeloid cells with regulatory properties, by production of Arginine 1;
Generating regulatory T cells by production of IL-10, prostaglandin E2 and TGF-beta;
Providing prostaglandin E2 (PGE2) as a substrate for the function of regulatory T cells in suppressing conventional T cells activation;
Participating in tissue repair by production of amphiregulin (Areg) ;
Production of metalloproteinase such as MMP9 and ADAMs such as ADAM33 with anti-inflammatory, pro-angiogenesis, tissue repair properties and increased thrombus resolution (MMP9);
Production of chitinase like proteins, such as chitinase 3-like-3, or products of equivalent genes in humans, with anti-inflammatory properties exerted on macrophages (M2 conversion) and activation of repair mechanisms and tissue regeneration;
Increasing fat tissue thermogenesis by TGF-beta-dependent increase in adrenergic innervation;
Production of bone morphogenetic protein 7 (BMP7) with increased neurone survival and neurogenesis;
Production of GDF11 with positive effect on axon remyelination;
Increase in angiogenesis by production of endothelial cell specific molecule 1 (ESM1) and potential effect on preventing graft rejection.

In addition, such cells express a variable number of surface molecules involved in suppressive functions, including TIGIT, DLL4 and CTLA2, as well as molecule such as Hobit important for tissue residency. Also there is the production of meteorin with increased differentiation of precursor cells for neurogenesis and axonal extension, of myocytes in muscle cell restoration and of chondrocytes in cartilage reconstruction.

Preferably, in the present invention (the method, epitope, CD4+ T cell, pharmaceutical composition or kit of parts), a MHC Class II epitope is identified and/or selected from the published documents and/or from bioinformatics studies. Since there are hundreds, if not thousands, MHC Class II epitope associated to an unwanted immune response, it is not possible nor useful to list them all. One skilled person can readily identify them and associate them to a disease, for instance, following the steps of identifying a disease associated to an unwanted immune response, selecting or identifying one or several peptide(s) (from the tissue and/or organ) associated to the disease, identifying therein, *in silico* and/or *in vitro,* one or several putative MHC Class II epitope associated to the unwanted immune response, thus preferably a MHC Class II epitope present in a protein from the tissue and/or organ affected by this unwanted immune response. An MHC Class II epitope can be identified in a peptide *in silico* by predicting the cleavage sites present in this peptide, then by predicting the peptides presented by MHC Class II, and, preferably, by predicting the recognition of the presented peptide by the T cell receptor (TCR).
Preferably, such MHC Class II epitope is thus found (deduced) in the tissue affected by the unwanted immune response.
Such MHC Class II epitope will then be modified by the addition of a so-called 'redox' motif. Such redox motif has been disclosed at least in WO 2008/017517.
Preferably, in the present invention (methods, MHC Class II epitope, CD4+ T cell, pharmaceutical composition or kit of parts), the redox motif comprises or consists essentially of the peptide sequence CXXC, preferably a redox motif selected from the group consisting of CPXC, CXPC, HCXXC, CXXCH, HCPXC, CPCXH, HCXPC and CXPCH, wherein C stands for cysteine or selenocysteine, X stands for any amino acid, P for proline and H for histidine.
The present invention will thus refer (i) to a MHC Class II epitope, to mean a part of a protein (deduced from a patient's tissue affected by an unwanted immune response) being loaded and presented by an antigen-presenting cell (APC), in order to be subsequently recognized by CD4+ (Foxp3 (-)) T lymphocytes and (ii) to a corresponding modified MHC Class II epitope, which is (substantially) the same epitope sequence, further comprising at least one redox motif, either immediately at a border (at the N- and/or the C-extremity of the MHC Class II epitope), or in its close proximity (e.g. with a linker of up to 7 amino acids).

A first aspect of the present invention is a (an *in vitro* or *ex vivo*) method to elicit CD4+ T cells with an immune suppressive function (for a specific target tissue) towards a given MHC Class II epitope and/or with tissue homeostasis restoration properties comprising the steps of: identifying a MHC Class II epitope associated to an unwanted immune disease to a mammal, preferably this MHC Class II epitope is identified from the patient's tissue (and/or organ) affected by this unwanted immune disease; of generating a modified MHC Class II epitope comprising at least one additional redox motif adjacent to the said MHC Class II epitope or separated to the said MHC Class II epitope by a linker of up to 7 amino acids, and of contacting *in vitro* CD4+ T cells (obtained from a patient) with the said modified MHC Class II epitope.
In this *in vitro* or *ex vivo* method, preferably, the CD4+ T cells are Foxp3 (-).
Advantageously, in this *in vitro* or *ex vivo* method these CD4+ T cells are contacted (*in vitro*) with an antioxidant (following the teachings of patent application EP19182807.8 filed on June 27, 2019) together with the modified MHC Class II epitope; this allows a more potent effect. Among the antioxidant are molecules compatible with the biology of cells, preferably also compatible with a pharmaceutical use, and strong enough to cleave disulfide bridges. A convenient antioxidant comprises -SH active moieties, such as a cysteine moiety, for instance (reduced) glutathione.

Preferably, this *in vitro* or *ex vivo* method further comprises the step of characterizing and/or of sorting and/or of isolating the CD4+ T cells (Foxp3(-)) for their expression of surface markers selected from the group consisting of TIGIT, DLL4 and CTLA2, and/or for their secretion of soluble factors selected from the group consisting of Interleukin (Il)-10, IL-13, IL-4, transforming growth factor (TGF) β-2, amphiregulin, arginase and meteorin; this allows to ensure the production and sorting of the right CD4+ T lymphocytes, which is particularly important in view of the plasticity of the CD4+ T lymphocytes. Such characterizing and/or sorting and/or isolating step can be performed after the contact with the modified MHC Class II epitope and/or before this step, such as a preliminary sorting of CD4+ lymphocyte, the measurement of the profile of the secreted cytokines (this can be done directly from a sample of the culture medium), the contact with the MHC Class II epitope, then the measurement of the profile of the secreted cytokines (to ensure a correct/improved expression profile). In this characterizing step, surface markers of such CD4+ T cells are advantageously determined by incubating cells with fluorescence-labelled specific antibodies and detection with FACS (Fluorescence-activated cell sorter). Expression of transcription factors is advantageously detected by either RNASeq or qPCR. The array of produced proteins is preferably evaluated by mass spectrometry. Epigenetic alterations are measured by the ATAC-Seq (assay for transposase-accessible chromatin). Secreted cytokines are preferably evaluated by multiplex and intracellular cytokines are preferably detected by incubation with specific labelled antibodies on permeated cells.
A related aspect of the present invention is the CD4+ T cells obtainable by this *in vitro* or *ex vivo* method, as well as their use as a medicament, advantageously for the treatment of an unwanted immune response and/or for use in tissue repair. Indeed, such engineered CD4+ T cells are now able to act specifically and locally on a tissue affected by an unwanted immune response allowing (i) the reduction of the unwanted immune response, such as inflammation, at the targeted tissue of a patient, which allows the normal metabolism to restart and (ii) the *in situ* production of soluble factors, which boosts the regeneration of the targeted tissue: the present invention is thus particularly potent for a local response and for tissue repair.
A related aspect is thus a modified MHC Class II epitope comprising at least one additional redox motif adjacent to a MHC Class II epitope from a specific target tissue or separated to the said MHC Class II epitope from a specific target tissue by a linker of up to 7 amino acids for use in the localized production of cytokines with a suppressive function (of the unwanted immune response) in a patient having developed an unwanted immune response towards a peptide (from a specific target tissue) bearing this MHC Class II epitope or bearing a substantially similar MHC Class II epitope. Indeed, when an unwanted immune response has been diagnosed to a patient, its cause can readily be determined, which is the peptide (from the affected tissue and/or organ) causing this unwanted immune response. Then, depending on the HLA typing of the patient, a (one or several) MHC Class II epitope can be determined (*in silico,* or because such epitope has already been found in other patients), then produced and grafted with this redox motif. The advantage of the present invention over the injection of cytokines to a patient are the localization of the effect to the affected tissue and/or organ and its longer duration: the CD4+ T lymphocytes elicited by such MHC Class II-redox peptide will be attracted/kept at the locus of the cognate unwanted immune response where they will produce the desired cytokines (predominantly, if not only, there), until the immune response has been turned-off.
Advantageously, this modified MHC Class II epitope allows for the conversion in a specific tissue of a patient of inflammatory macrophage into non-inflammatory macrophage, preferably in the adipose tissue.
Therefore, another aspect of the present invention is a modified MHC Class II epitope comprising at least one additional redox motif adjacent to the said MHC Class II epitope, or separated to the said MHC Class II epitope by a linker of up to 7 amino acids, for use in tissue repair in a patient suffering from an unwanted immune disease, or from an inflammatory disease caused by an unwanted immune response, towards a (naturally-occurring) peptide bearing this MHC Class II epitope or bearing a substantially similar MHC Class II epitope.
Another related aspect of the present invention is a kit of parts comprising a modified MHC Class II epitope comprising at least one additional redox motif adjacent to the said MHC Class II epitope or separated to the said MHC Class II epitope by a linker of up to 7 amino acids for use in the treatment of an unwanted immune response to a (naturally-occurring) peptide comprising the said MHC Class II epitope (and causing this unwanted immune response) and means to monitor the activation of Foxp3(-) CD4+ T cells. This will allow a better monitoring, at the patient's level, of the response of the immune system.
Preferably, in this kit of parts, the means to monitor the activation of Foxp3(-) CD4+ T cells are means for measuring the expression by these Foxp3(-) CD4+ T cells of surface markers selected from the group consisting of TIGIT, DLL4 and CTLA2, and/or means to monitor the secretion by these Foxp3(-) CD4+ T cells of soluble factors selected from the group consisting of Interleukin (Il)-10, IL-13, IL-4, transforming growth factor (TGF) β-2, amphiregulin, arginase and meteorin.
Preferably, this kit of parts further comprises an antioxidant compound (for instance glutathione or similar pharmaceutically acceptable compounds having the capacity to reduce disulphide bridges, following the teachings of patent application EP19182807.8 filed on June 27, 2019) to be administered to a patient together with the modified MHC Class II epitope.
Another related aspect of the present invention is a modified MHC Class II epitope comprising at least one additional redox motif adjacent to such MHC Class II epitope or separated to such MHC Class II epitope by a linker of up to 7 amino acids for use in eliciting Foxp3(-) CD4+ T cells (specific for this MHC Class II epitope) expressing surface markers selected from the group consisting of TIGIT, DLL4 and CTLA2, and/or for their secreting soluble factors selected from the group consisting of Interleukin (Il)-10, IL-13, IL-4, transforming growth factor (TGF) β-2, amphiregulin, arginase and meteorin.
Another related aspect is a kit of parts comprising:
a gene editing system with a specific nuclease (such as CRISPR/Cas (Cas9, Cpf1, Csm and the like), but also TALEN or any other gene editing system based on the administration of an exogenous nuclease specifically cleaving DNA so as to subsequently introduce a desired modification at a targeted genomic locus) or a nucleic acid molecule encoding the said specific nuclease and
a fragment of the said specific nuclease being a MHC Class II epitope further comprising a redox motif adjacent to such fragment of this specific nuclease or separated to such fragment of this specific nuclease by a linker of up to 7 amino acids.
This kit is advantageously for use in the treatment of a genetic disease. Indeed, the administration to a patient of an exogenous specific nuclease, usually of a prokaryotic origin and/or an artificial construct, will be problematic because of the immune response towards this nuclease. The specific downregulation of the resulting unwanted immune response allowed by the present invention paves the way of this new approach.

### Examples

### Example 1

### Neuromyelitis optica (NMO)

NMO is a severe inflammatory disease of the central nervous system (CNS) accompanied by antibodies to aquaporin 4 (AQP4). Lesions localize to the spinal cord and the anterior visual pathway wherein the concentration of astrocyte AQP4 is the highest.

The lesions are dependent on the production of anti-AQP4 antibodies in the periphery, yet such antibodies have to cross the blood-brain barrier (BBB), which is dependent on inflammation as provided by specific CD4+ T cells. *In situ,* antibodies activate the complement pathway at the surface of astrocytes, leading to the formation of the lytic complex and cell cytotoxicity.

There is no animal model which reconstitutes the whole of human disease, but mice develop a similar pathology by accumulation of AQP4-specific CD4+ T cells of the TH17 subtype, leading to opening of the BBB and local inflammation, in the absence of anti-AQP4 antibodies.

The C57BL/6 (H-2b) strain is the elective animal model as its natural repertoire of CD4+ T cells contains cells activable by immunisation with AQP4 epitopes whose sequences are entirely superposable to those activating human CD4+ T cells. Besides, the homology between human and mouse AQP4 is 93%. However, due to natural tolerance to AQP4, it remains difficult to generate T cells with a pathogenic potential. Therefore, C57BL/6 mice lacking expression of AQP4 (AQP4 KO) are used.

A peptide encompassing amino acid residues 21-38 of AQP4 (SEQ ID NO:1)
SIMVAFKGVWTQAFWKAV (core epitope underlined)
is used at 100 µg emulsified in complete Freund's adjuvant to immunize AQP4 KO C57BL/6 mice in the footpad, followed by 3 injections of 100 µg in incomplete Freund's adjuvant at 10-days intervals. Four weeks after the last injection, the mice are sacrificed and splenocytes are prepared. After separation of CD4+ T cells from the bulk splenocytes, lymphocytes specific for peptide of SEQ ID NO:1 are isolated by incubation with magnetic beads coated with class II (H2b) molecules loaded with peptide of SEQ ID NO:1.

AQP4-specific CD4+ T cells are then phenotyped and found to belong to the Th17 subtype.

Such cells are then administered by IV injection in wildtype C57BL/6 mice (10⁶ cells per mouse). Ten days afterwards, mice are sacrificed and the spinal cord isolated. Infiltration with inflammatory cells, including AQP4-specific CD4+ T cells is observed in close proximity to astrocytes.

A similar experiment is carried out in AQP4 KO C57BL/6 mice immunized with the same peptide, but containing a redox motif (in bold) in its flanking residues (SEQ ID NO:2), and with alum instead of CFA/IFA

### HCPYCSIMVAFKGVWTQAFWKAV

CD4+ T cells obtained from the spleen are characterized by markers such as TIGIT and CTLA2 and produce PGE2 (prostaglandin E2) and IL-10.

In a control experiment, 10⁶ cells are administered by IV injection in wildtype C57BL/6 mice. The spinal cord shows no inflammatory lesions or T lymphocytes, indicating that the population of CD4+ T cells obtained by immunization with peptide of SEQ ID NO:2 does not induce an inflammatory reaction required to permeate the BBB and allow the egress of T cells in the CNS.

Next, to determine whether CD4+ T cells obtained by immunisation with peptide of SEQ ID NO:2 could suppress pathogenic CD4+ T cells obtained by immunisation with peptide of SEQ ID NO:1, mixed cultures were performed with antigen-presenting cells loaded with peptide of SEQ ID NO:1. For such *in vitro* experiments, the two CD4+ T cell populations were obtained from C57BL/6 mice expressing either the Thy1.1 or the Thy1.2 surface phenotypic marker, identified by specific antibodies.

Dendritic cells converted from peripheral blood monocytes were used as antigen-presenting cells. Thus, 50x10⁶ cells were incubated with 10 µg of peptide of SEQ ID NO:1 for 16 h at room temperature, washed and then incubated with a 1/1 mixture of Thy1.1 CD4+ T cells obtained by immunisation with peptide of SEQ ID NO:1 and Thy1.2 CD4+ T cells obtained by immunisation with peptide of SEQ ID NO:2. The phenotype of each CD4+ T cell population was then evaluated after 7 days of culture.

It is shown that Thy1.1 CD4+ T cells have lost their phenotypic characteristics of Th17 cells and show no proliferation.

It is therefore concluded that CD4+ T lymphocytes stimulated with a cognate epitope containing a redox motif in its flanking residues suppress the population of cells stimulated with the cognate epitope with no redox motif, but show no evidence of cytotoxicity.

### Example 2

### Insulin-dependent diabetes

Insulin-dependent; or type 1 diabetes is characterized by the gradual loss of pancreatic beta cells due to a combination of *in situ* inflammation and auto-immune response towards a number of antigens, including glutamic acid decarboxylase (GAD65). Several lines of evidence indicate that intra-islet accumulation of GAD65 specific CD4+ and CD8+ T cells is associated with diabetes. A therapy which would lead to the generation of suppressive CD4+ T cells specific for GAD65 would result in resolution of inflammation and elimination of immune cells (CD4+ and CD8+ T cells) from pancreatic islets, independently of their precise antigen specificity.

Non-obese diabetes (NOD) mice are the most used animal model for diabetes due to the spontaneous development of insulitis, hyperglycaemia and diabetes in a context wherein both inflammation and auto-immunity play determining roles. The local lesion, insulitis, starts accumulating after a few weeks, so that therapeutic interventions should be considered as suppressive rather than preventive.

Six-weeks old female NOD mice are immunized with a peptide made of a class II-restricted T cell epitope of GAD65 (319-333: ILEVKQKGFVPFLVS, SEQ ID NO:3) or with the same peptide containing a redox motif (bold figures) in its flanking residues

### HCYPCILEVKQKGFVPF (SEQ ID NO:4)

Immunisation is carried out using 100 µg of peptide of SEQ ID NO:4 in aluminum hydroxide, four times at a week interval. Control mice receive peptide of SEQ ID NO:3 according to the same injection schedule.

Mice are then sacrificed at week 15, the pancreas is isolated and evaluated by histology to identify insulitis.

It is shown that mice immunized with peptide of SEQ ID NO:4 containing the redox motif conserve a large majority (≥70%) of islets with no sign of inflammation or cellular infiltrate, whilst the reverse is shown for mice immunized with peptide of SEQ ID NO:3.

This indicates that the generation of CD4+ T cells by immunisation with peptide of SEQ ID NO:4 is sufficient as to eliminate immune cells accumulating in islets, independently of their antigenic specificity.

In *in vitro* cultures, it is shown that CD4+ obtained from the spleen of mice immunized with peptide of SEQ ID NO:3 (and labelled with CFSE) are minimally reduced in numbers (circa 5%) by co-culture with CD4+ T cells obtained from the spleen of mice immunized with peptide of SEQ ID NO:4. However, CFSE-labeled cells recovered from such co-culture have lost their capacity to proliferate upon presentation of their cognate epitope (SEQ ID NO:3). This indicates that cells obtained from mice immunized with the redox-containing epitope exert a suppressive activity, yet little or any cytotoxicity on cells expanded with the unmodified epitope.

### Example 3

### Multiple sclerosis

Multiple sclerosis is a chronic autoimmune disease showing accumulation of immune and inflammatory cells into the white zone of the CNS. A major factor in the disease is demyelination and exposure to axon destruction, leading to progressive sensitive and motor degradation. Elimination of the autoimmune reaction towards myelin is therefore an unattained, yet hoped-for expectation.

Myelin is made of several constituents, including major basic protein (MBP). Autoimmune reactivity towards MBP is well described and a predictive model is at hand to assess the impact of various formats of immunotherapy.

C57BL/6 female mice are treated with an injection of 100 µl containing 200pg of peptide encompassing amino acid residues 35 to 47 of MBP, namely:
TGILDSIGRFFSG (SEQ ID NO:5)
and 100 µl of complete Freund's adjuvant. In addition, 200 µg of pertussis toxin is administered intraperitoneally on days 0 and 2 to permeate the blood-brain barrier.

Signs associated with induction of encephalomyelitis are observed after 6 days, including paresis and flaccid tail, which develop over a period of several weeks. One month after the date of disease induction, the spinal cord is examined for inflammatory infiltrates. Tissues are fixed in formaldehyde and stained with hematoxilin-eosin for cell counting. T cells are identified using an anti-CD3 antibody. Demyelination is detected by staining with Luxol Fast Blue (LFB). It is observed that all mice present both significant infiltrates including T lymphocytes and signs of demyelination.

To establish that cells of the present invention exert a suppressive effect on pathogenic T cells, two group of C57BL/6 female mice was treated by 4 weekly subcutaneous injections of 100 µg of peptide
**HCYPC**TGILDSIGRFFSG (SEQ ID NO:6)
corresponding to peptide of SEQ ID NO:5 but containing a redox motif (in bold). These injections were made with a formulation containing aluminum hydroxyde as adjuvant. To note, no sign of disease were observed in such mice.

In a first group, mice were sacrificed on day 35 after the start of the immunization schedule, and splenocytes prepared by isolation of CD4+ T lymphocytes. The latter were then cultured in the presence of antigen-presenting cells loaded with peptide of SEQ ID NO:5. After 7 days of culture, the phenotype and cytokine production of CD4+ T cells was evaluated. It is shown that such cells express the transcription factor ROR-α (NR1F1) and BMP7 (bone morphogenetic protein 7) and produce GDF11, conferring them with anti-inflammatory and tissue repair properties, but no expression of perforin.

A second group of mice immunized with peptide of SEQ ID NO:6 was then treated as in the positive control group, namely peptide of SEQ ID:5 with complete Freund's adjuvant and pertussis toxin. This group of mice did only manifest minor and transient signs of disease, with complete recovery within less than one week. Similarly, absent or minor T lymphocyte infiltrates were observed in the spinal cord and no obvious sign of demyelination was observed after LFB staining.

It was therefore concluded that CD4+ T cells obtained after immunisation with peptide of SEQ ID NO:6 containing a redox motif showed a suppressive function of pathogenic T cells elicited by immunisation with peptide of SEQ ID NO:5.

### Example 4

### Nucleases

Nucleases are essential for gene editing. However, nucleases originate from bacteria such as those of the *Streptococcus* or *Staphylococus* species to which everyone is exposed. As a consequence virtually every individual carries specific antibodies and T cells, which prohibit the use of such nucleases. Eliminating such reactivity without affecting the overall defence towards bacteria is therefore much desirable.

Balb/c mice kept under open conditions (not pathogen-free) show specific antibodies and CD4+ T cells towards Staph. aureus nuclease. Splenocytes are obtained from such mice to prepare CD4+ T cells. Such cells are cultured and expanded in the presence of antigen-presenting cells loaded with either peptide of sequence
NTHEQHLRKS (SEQ ID NO:7)
or peptide of sequence
**HCYPC**HNTHEQHLRKS (SEQ ID NO:8)
containing a redox motif (in bold).

After 2 cycles of stimulation, the phenotype and cytokine production of cells are examined. Cells obtained by stimulation with peptide of SEQ ID NO:7 present the characteristics of Th1 cells and produce interferon gamma (IFN-γ). By contrast, cells obtained from cultures in the presence of peptide of SEQ ID NO:8 produce IL-4, IL-10, IL-13 and amphiregulin (AREG), and show surface expression of TIGIT and BLIMP-1.

Cells obtained by stimulation with peptide of SEQ ID NO:7 were labelled with CFSE and cultured together with cells obtained with peptide of SEQ ID NO:8 at a 1 to 1 ratio on antigen-presenting cells loaded with peptide of SEQ ID NO:7.

After 7 days of coculture, the proliferation of cells was measured by CFSE dilution. It is shown that cells obtained by exposure to peptide of SEQ ID NO:7 do not proliferate in the presence of cells obtained by exposure to peptide of SEQ ID NO:8. Further evaluation of cells obtained with peptide of SEQ ID NO:8 show an unchanged phenotype, indicating that the properties acquired by exposure to an epitope containing a redox motif are not reversible.

It is therefore concluded that a redox motif when attached to a MHC class II epitope elicit a population of CD4+ T cells with suppressive yet not cytotoxic properties and that such properties are maintained under natural conditions of exposure to the same class II epitope.

## Claims

1. An *in vitro* or *ex vivo* method to elicit CD4+ T cells with an immune suppressive function towards a given MHC Class II epitope present in a specific target tissue and/or to elicit CD4+ T cells harbouring homeostasis restoration properties for a specific target tissue, the said *in vitro* or *ex vivo* method comprising the steps of:
identifying a MHC Class II epitope from the said tissue;
generating a modified MHC Class II epitope comprising at least one additional redox motif adjacent to the said MHC Class II epitope or separated to the said MHC Class II epitope by a linker of up to 7 amino acids, and
contacting *in vitro* CD4+ T cells with the said modified MHC Class II epitope,
wherein the said target tissue is affected by an unwanted immune disease.

2. The method of claim 1, wherein the CD4+ T cells are contacted with a pharmaceutically-acceptable antioxidant together with the modified MHC Class II epitope.

3. The method according to any one of the preceding claims 1 to 2, further comprising the step of characterizing and/or of sorting and/or of isolating the CD4+ T cells (Foxp3(-)) for their expression of surface markers selected from the group consisting of TIGIT, DLL4 and CTLA2, and/or for their secretion of soluble factors selected from the group consisting of Interleukin (Il)-10, IL-13, IL-4, transforming growth factor (TGF) β-2, amphiregulin, arginase and meteorin.

4. CD4+ T cells obtainable by the method according to any one of the preceding claims 1 to 3, being preferably for use as a medicament, more preferably for use in the treatment of an unwanted immune response and/or for use in tissue repair.

5. A modified MHC Class II epitope comprising at least one additional redox motif adjacent to a MHC Class II epitope, or separated to the said MHC Class II epitope by a linker of up to 7 amino acids,
for use in the localized production of cytokines with a suppressive function in a tissue of a patient having developed an unwanted immune response towards a peptide bearing the said MHC Class II epitope or bearing a substantially similar MHC Class II epitope.

6. The modified MHC Class II epitope of claim 5 being for the conversion in the tissue of the patient of inflammatory macrophages into non-inflammatory macrophages, preferably in the adipose tissue.

7. A modified MHC Class II epitope comprising at least one additional redox motif adjacent to a MHC Class II epitope, or separated to the said MHC Class II epitope by a linker of up to 7 amino acids,
for use in tissue repair in a patient suffering from an unwanted immune disease towards a peptide bearing the said MHC Class II epitope, or bearing a substantially similar MHC Class II epitope.

8. A kit of parts comprising:
a modified MHC Class II epitope comprising at least one additional redox motif adjacent to a MHC Class II epitope, or separated to the said MHC Class II epitope by a linker of up to 7 amino acids, and
means to monitor the activation of (Foxp3(-)) CD4+ T cells,
wherein the said MHC Class II epitope is expressed in a patient's tissue and/or organ affected by the said unwanted immune response, and
wherein the said kit of parts is for use in the treatment of the unwanted immune response towards the peptide bearing the said MHC Class II epitope.

9. The kit of parts of claim 8, wherein the means to monitor the activation of (Foxp3(-)) CD4+ T cells are means for measuring the expression by the said (Foxp3(-)) CD4+ T cells of surface markers selected from the group consisting of TIGIT, DLL4 and CTLA2, and/or means to monitor the secretion by the said (Foxp3(-)) CD4+ T cells of soluble factors selected from the group consisting of Interleukin (Il)-10, IL-13, IL-4, transforming growth factor (TGF) β-2, amphiregulin, arginase and meteorin.

10. The kit of parts of claims 8 or 9 further comprising an antioxidant compound to be administered to a patient together with the modified MHC Class II epitope.

11. The modified MHC Class II epitope according to any one of the preceding claims, wherein the unwanted immune response is inflammation.

12. A modified MHC Class II epitope comprising at least one additional redox motif adjacent to a MHC Class II epitope, or separated to the said MHC Class II epitope by a linker of up to 7 amino acids,
for use in eliciting (Foxp3(-)) CD4+ T cells expressing surface markers selected from the group consisting of TIGIT, DLL4 and CTLA2, and/or secreting soluble factors selected from the group consisting of Interleukin (Il)-10, IL-13, IL-4, transforming growth factor (TGF) β-2, amphiregulin, arginase and meteorin,
the said CD4+ T cells being specific for the said MHC Class II epitope, the said MHC Class II epitope being expressed in a patient's tissue and/or organ affected by an unwanted immune response.

13. A kit of parts comprising
a gene edition system with a specific nuclease or a nucleic acid molecule encoding the said specific nuclease, and
a peptide fragment of the said specific nuclease being a MHC Class II epitope, the said peptide fragment further comprising a redox motif adjacent to the said fragment of the said specific nuclease or separated to the said fragment of the said specific nuclease by a linker of up to 7 amino acids.

14. The kit of claim 13 for use as a medicament, preferably for use in the treatment of a genetic disease and/or in the treatment and/or the prevention of a viral disease and/or in the treatment of cancer.

15. The method, MHC Class II epitope, CD4+ T cell, pharmaceutical composition or kit of parts according to any one of the preceding claims wherein the redox motif comprises or consists essentially of the peptide sequence CXXC, preferably a redox motif selected from the group consisting of CPXC, CXPC, HCXXC, CXXCH, HCPXC, CPCXH, HCXPC and CXPCH, wherein C stands for Cysteine or selenocysteine, X stands for any amino acid, P for proline and H for histidine.


SEQUENCE LISTING

<110>  Equaly

<120>  CD4redox

<130>  pbf1772EP00

<160>  8

<170>  PatentIn version 3.5

<210>  1
<211>  18
<212>  PRT
<213>  Artificial Sequence

<220>
<223>  AQP4 epitope


<220>
<221>  MISC_FEATURE
<222>  (4)..(15)
<223>  core epitope

<400>  1

Ser Ile Met Val Ala Phe Lys Gly Val Trp Thr Gln Ala Phe Trp Lys
1               5                   10                  15


Ala Val




<210>  2
<211>  23
<212>  PRT
<213>  Artificial Sequence

<220>
<223>  AQP4redox


<220>
<221>  MISC_FEATURE
<222>  (9)..(20)
<223>  core epitope

<400>  2

His Cys Pro Tyr Cys Ser Ile Met Val Ala Phe Lys Gly Val Trp Thr
1               5                   10                  15


Gln Ala Phe Trp Lys Ala Val
            20


<210>  3
<211>  15
<212>  PRT

<213>  Artificial Sequence

<220>
<223>  GAD65 epitope

<400>  3

Ile Leu Glu Val Lys Gln Lys Gly Phe Val Pro Phe Leu Val Ser
1               5                   10                  15


<210>  4
<211>  17
<212>  PRT
<213>  Artificial Sequence

<220>
<223>  GAD65redox

<400>  4

His Cys Tyr Pro Cys Ile Leu Glu Val Lys Gln Lys Gly Phe Val Pro
1               5                   10                  15


Phe




<210>  5
<211>  13
<212>  PRT
<213>  Artificial Sequence

<220>
<223>  MBP

<400>  5

Thr Gly Ile Leu Asp Ser Ile Gly Arg Phe Phe Ser Gly
1               5                   10


<210>  6
<211>  18
<212>  PRT
<213>  Artificial Sequence

<220>
<223>  MBPredox

<400>  6

His Cys Tyr Pro Cys Thr Gly Ile Leu Asp Ser Ile Gly Arg Phe Phe
1               5                   10                  15


Ser Gly



<210>  7
<211>  10

<212>  PRT
<213>  Artificial Sequence

<220>
<223>  Sa Nuclease

<400>  7

Asn Thr His Glu Gln His Leu Arg Lys Ser
1               5                   10


<210>  8
<211>  16
<212>  PRT
<213>  Artificial Sequence

<220>
<223>  Sa Nucleaseredox

<400>  8

His Cys Tyr Pro Cys His Asn Thr His Glu Gln His Leu Arg Lys Ser
1               5                   10                  15